(19) Europäisches Patentamt
European Patent Office
Office européen des brevets

(11) Publication number: **0 143 757**
**A2**

(12) # EUROPEAN PATENT APPLICATION

(21) Application number: **84850263.9**

(22) Date of filing: **10.09.84**

(51) Int. Cl.⁴: **C 07 D 211/22**, A 61 K 31/445

(30) Priority: **30.09.83 SE 8305362**

(43) Date of publication of application: **05.06.85**
**Bulletin 85/23**

(84) Designated Contracting States: **AT BE CH DE FR GB IT LI LU NL SE**

(71) Applicant: **Astra Läkemedel Aktiebolag, Strängnäsvägen 44, S-151 85 Södertälje (SE)**

(72) Inventor: **Arvidsson, Folke Lars-Erik, Seminariegränd 3, S-752 28 Uppsala (SE)**

(72) Inventor: **Carlsson, Per Arvid Emil, Torild Wulffsgatan 50, S-413 19 Göteborg (SE)**
Inventor: **Hacksell, Uli Alf, Tjudervägen 16, S-752 47 Uppsala (SE)**
Inventor: **Hjorth, John Stephan Mikael, Blavalsgatan 7A, S-414 75 Göteborg (SE)**
Inventor: **Johansson, Anette Margareta, Blodstensvägen 7, S-752 44 Uppsala (SE)**
Inventor: **Lindberg, Per Lennart, Knapehall 64, S-436 00 Askim (SE)**
Inventor: **Nilsson, John Lars Gunnar, Tullinge Strand 30B, S-146 00 Tullinge (SE)**
Inventor: **Sanchez, Domingo, Snipasvägen 24, S-448 00 Floda (SE)**
Inventor: **Svensson, Kjell Anders Ivan, Torild Wulffsgatan 4, S-413 19 Göteborg (SE)**
Inventor: **Wikström, Hakan Vilhelm, Gibsons väg 25, S-433 61 Partille (SE)**

(74) Representative: **Wurm, Bengt Runio et al, Patent and Trade Mark Department AB ASTRA, S-151 85 Södertälje (SE)**

(54) **Novel substituted phenylazacycloalkanes, process for preparation and pharmaceutical preparations for such compounds.**

(57) A compound of the formula

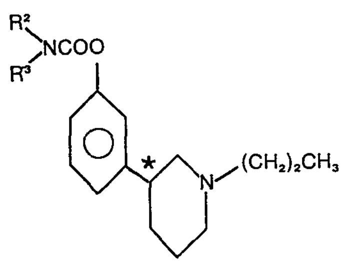

(I)

wherein $R^2$ is n-propyl, isopropyl, tert.-butyl or one of the groups

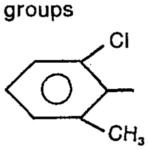

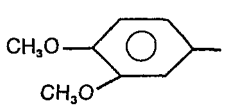

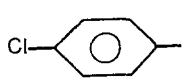

and $R^3$ is hydrogen; or $R^2$ and $R^3$ are each a n-propyl, isopropyl or tert.-butyl group; or $R^2$ and $R^3$ together form the group $-(CH_2)_5-$, as the base or a pharmaceutically acceptable acid addition salt thereof, processes for preparation of such compounds and pharmaceutical preparations and methods of treatment employing a such compound. The compound is useful for treatment of disorders in the central nervous system.

EP 0 143 757 A2

Novel substituted phenylazacycloalkanes, processes for preparation and pharmaceutical preparations for such compounds

DESCRIPTION

Technical field

The present invention is related to novel substituted phenylazacycloalkanes, to processes for preparing such compounds as well as to pharmaceutical preparations thereof and methods of treatment employing such compounds.

An object of the invention is to provide compounds for therepeutic use, expecially having a therapeutic activity in the central nervous system.

Background Art

EP-A1-0030526 and Hacksell et al. in J. Med. Chem., vol. 24, p. 1475-1482 (1981) describe compounds of the formula

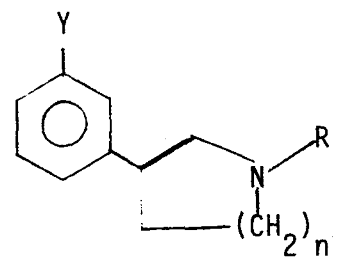

wherein n is 1 or 2, Y is OH, $R^1COO$, $R^2R^3NCOO$- or $R^4O$ whereby $R^1$ is an alkyl group having 1-5 carbon atoms or a possibly substituted phenyl group, $R^2$ is an alkyl group having 1-5 carbon atoms, a phenethyl, benzyl or phenyl group, $R^3$ is H or an alkyl group having 1-5 carbon atoms, and $R^4$ is an allyl or benzyl group, and R is an alkyl group having 1-5 carbon atoms, a hydroxyalkyl, dimethylaminoalkyl or methylthioalkyl group having 2-6 carbon atoms in the alkyl part and having the heteroatom bound in a position other than the 1 position, or an alkenyl group having 3-5 carbon atoms other than a 1-alkenyl group, as bases and pharmaceutically acceptable acid addition salts thereof, which compounds

are potent neuropharmacological agents. Thus said compounds are active as presynaptic dopamine receptor agonists when administered to animals including man. Said compounds are thus useful for treatment of disorders in the central nervous system, expecially psychotic disorders in man.

In Acta Pharmaceutica Suecica Suppl. 1983:1 p. 130-137 and 145-153 the pharmacological properties of the (-) and (+) enantiomers of the compound

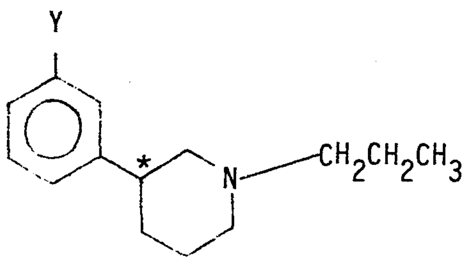

are described.

In European Patent Application No. 83850084.1 expected to be published after September 30, 1983, enantiomers of compounds of the formula

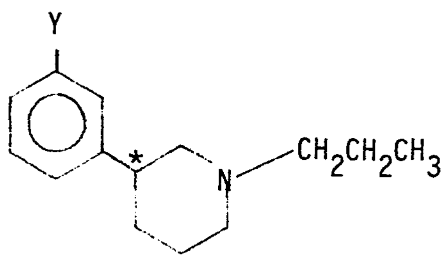

are described wherein Y is OH, $R^1COO$, $R^2R^3NCOO$ or $R^4O$. In compounds wherein Y is $R^2R^3NCOO$ $R^2$ is hydrogen, an alkyl group having 1-5 carbon atoms, a phenethyl, benzyl or phenyl group which may be mono- or disubstituted in the aromatic part with a methyl, methoxy, hydroxy, nitro or cyano group or a halogen, $R^3$ is H, an alkyl group having 1 to 5 carbon atoms or a phenyl group or $R^2$ and $R^3$ together with the nitrogen atom form a 5, 6 or 7 membered ring that may contain 1 to 3 double bonds and/or 1 or 2 further heteroatoms selected from N, O and S. Specifically described in either of the two European patent applications above is i.a. a compound wherein Y is

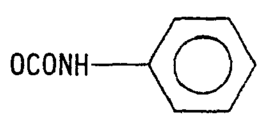

## Disclosure of Invention

According to the present invention it has been found that compounds of the formula

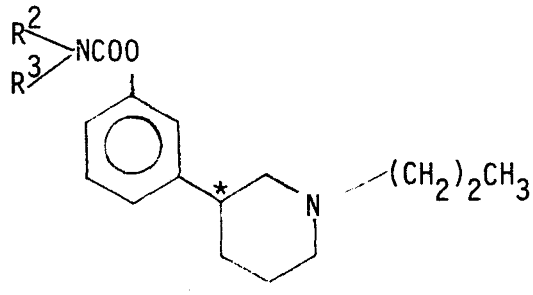

I

wherein $R^2$ is n-propyl, isopropyl, tert. butyl or one of the groups

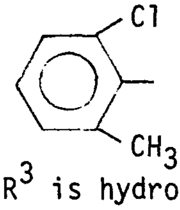 , 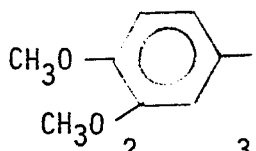 , 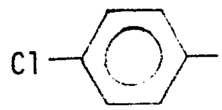

and $R^3$ is hydrogen; or $R^2$ and $R^3$ are each a n-propyl, isopropyl or tert. butyl group; or $R^2$ and $R^3$ together form the group $-(CH_2)_5-$ as bases and pharmaceutically acceptable acid addition salts thereof possess unexpected valuable therapeutical properties.

Symbols for numbers, atoms or groups referred to below have the broadest meaning previously assigned unless specified otherwise.

Both organic and inorganic acids can be employed to form non-toxic pharmaceutically acceptable acid addition salts of the compounds of this invention. Illustrative acids are sulfuric, nitric, phosphoric, hydrocloric, citric, acetic, lactic, tartaric, pamoic, ethanedisulfonic, sulfamic, succinic, cyclohexylsulfamic, fumaric, maleic and benzoic acid. These salts are readily prepared by methods known in the art.

The compounds of the invention contain an asymmetric carbon atom in the heterocyclic ring moiety. The therapeutic properties of the compounds may to a greater or lesser degree be ascribed to either or both of the two enantiomers occurring. Thus the pure enantiomers as well as mixtures thereof are within the scope of the invention.

In particular, the most valuable therapeutical properties have been found to reside in compounds having S configuration at the asymmetric carbon atom (*) which thus constitute a preferred embodiment of the invention.

Methods of Preparation

The compounds of the invention may be obtained by one of the following methods constituting a further aspect of the invention.

a) A compound of the formula

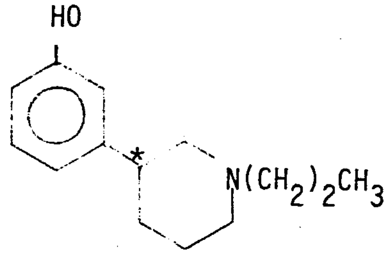

II

may be converted into a compound of formula I by treating the firstmentioned compound with an appropriate carbamic acid derivative

$R^2R^3NCOX$ or aminoformaldehyde $R^2R^3NCOH$ or isocyanate $R^2NCO$ or isocyanide $R^2N\rightleftarrows C$,

whereby X represents a leaving group such as a halogen preferably Cl or Br or a metal sulfite $SO_3$ Me or the group $R^2R^3N$

The reaction with a carbamoyl halide or isocyanate is done in the presence of a base such as triethylamine or pyridine or an acid such as $H_2SO_4$ or $CF_3COOH$. The reaction with the isocyanide is done in the presence of a halogen or halogen generator such as $Br_2$, $Cl_2$ or N-bromosuccinimide. The reaction with the aminoformaldehyde may be carried out in the presence of an oxidant such as lead tetraacetate.

The isocyanate $R^2NCO$ may be added as such or formed *in situ* as described below.

Compound II may be present in the form of the phenol or a metal salt thereof e.g. the sodium salt.

When X represents the group $R^2R^3N$ the reaction is carried out at an elevated pressure and temperature

b) A compound of the formula

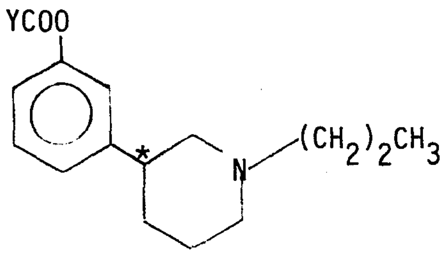

III

wherein Y is a leaving group obtainable by reaction of phosgene or a phosgene derivative with a phenol of formula II above, may be reacted with an amine of the formula

$$R^2R^3NH \qquad IV$$

to the formation of a compound of formula I. Examples of phosgene derivatives are

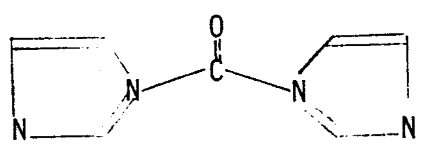 , 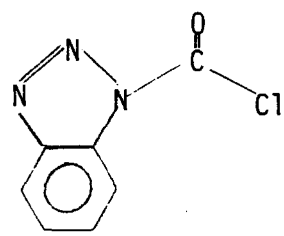 and 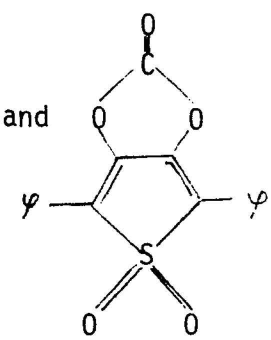

wherein $\varphi$ is a phenyl group. Examples of groups Y are thus chlorine or a group derived from the phosgene derivative employed.

c) A compound of the formula

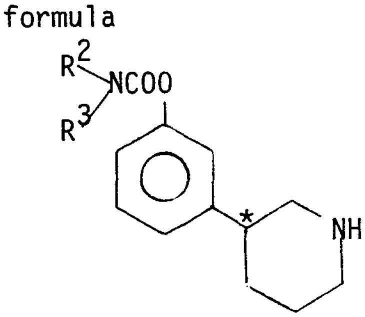

V

may be converted into a compound of formula I by alkylation of the nitrogen atom with an appropriate alkylating agent. Thus the starting compound may be treated with a n-propyl halide or tosylate $RX^1$, wherein $X^1$ represents Cl, Br, I or $OSO_2$ 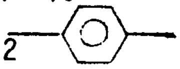 $CH_3$ in an organic solvent such as acetonitrile or acetone and in the presence of a base such as $K_2CO_3$ or NaOH, or the starting compound may be treated with a n-propionic acid $NaBH_4$ complex.

When possible and desired a reactant may be inserted as a salt, such as a phenolic metal salt or a quaternary ammonium acid addition salt, in the place of the phenol or amine described above.

Free bases formed may subsequently be converted into their acid addition salts, and acid addition salts formed may subsequently be converted into the corresponding bases or other acid addition salts.

To obtain a pure enantiomer of a compound of formula I, a racemic mixture partly enriched on one of the enantiomers of a compound of the formula I may subsequently be subjected to enantiomeric separation to obtain the desired enantiomer of compound I. This may be done by methods known in the art. These methods include recrystallization of diastereomeric salts with pure enantiomers of acids such as tartaric acid, O,O-dibenzoyltartaric acid, mandelic acid and camphor-10-sulphonic acid.

The above methods are equally useful for preparing compounds described in European Patent Application No. 83850084.1 as presented above wherein Y is $R^2R^3NCOO$. A further embodiment of the present invention is thus preparation of compounds described in the above-identified patent application by one of the above methods, to the extent such methods are not previously disclosed in European Patent Application No. 83850084.1 or a counterpart thereof in any country.

<u>Preparation of starting materials</u>

Starting materials for the methods of preparation described above may be obtained by several methods known in the art and/or described below.

Thus, the compound of formula II and the compound of formula V above are obtainable as described in EP-A1-0030526. To obtain said compound in pure enantiomeric form enantiomer separation is carried out on the racemic compound or a precursor thereof as further described in European Patent Application No. 83850084.1.

The isocyanate employed by method a) may be formed in situ by one of the reactions

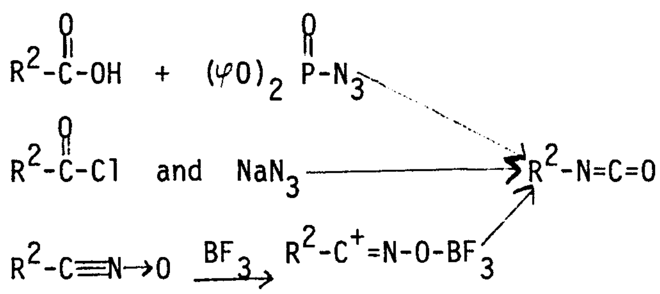


whereby φ represents phenyl.

Pharmaceutical preparations

Pharmaceutical preparations of the compounds of the invention constitute a further aspect of the invention. For such preparations reference is made to pages 23 to 25 of EP-A1-0030526.

In therapeutical treatment the suitable daily doses of the compounds of the invention are 200-10000 mg for oral application, preferentially 1000-6000 mg, and 1-1000 mg for parenteral application, preferentially 50-500 mg.

Working examples

The following examples will further illustrate the invention. Temperatures are in °C.

Example 1. A mixture of (-)N-propyl-3-(hydroxyphenyl)piperidine base (3.0 g; 0.0137 mol) in toluene (40 ml) and propylisocyanate (2.34 g; 0.0274 mol) was refluxed for 4 hours. The solution was evaporated and the residues were dissolved in ether. HCl-saturated ether was added giving white crystals. Filtration and recrystallisation from acetonitrile gave the desired compound, (-)N-propyl-3-[3-(propylcarbamoyloxy)-phenyl]piperidine. Yield: 75% m.p. 186-188°C $[\alpha]_D^{20}$ = (-)7.85° [C = 2.1; MeOH]

Example 2. (-)N-propyl-3-[3-(diisopropylcarbamoyloxy)phenyl]piperidine was prepared in analogy with the method for (-)N-propyl-3-[3-(piperidine-carbamoyloxy)phenyl]piperidine according to Example 3 from the corresponding diisopropylcarbamoylchloride. M.p. 193-195°C $[\alpha]_D^{20}$ (-)5.59°

Example 3. A solution of piperidinecarbonylchloride (4.03 g; 0.0273 mol) and (-)N-propyl-3-(hydroxyphenyl)piperidine (2.0 g; 0.0091 mol) in pyridine (25 ml) was stirred at 60°C for 4 hours. The pyridine was evaporated carefully. The residue was washed with ether and tetrahydrofuran. Recrystallization from acetonitrile afforded the pure product (-)N-propyl-3(3-piperidinecarbonyloxyphenyl)piperidine. Yield: 57% m.p. 209-210°C $[\alpha]_D^{20}$ = (-)8.3° [C = 2.1; MeOH].

Example 4. (-)N-propyl-3-[3-(3,4-dimethoxyphenylcarbamoyloxy)phenyl]-piperidine was prepared in accordance with Example 1. M.p. 183-184°C, $[\alpha]_D^{20}$ = 1.28°

Example 5. (-)N-propyl-3-[3-(p-chlorophenylcarbamoyloxy)phenyl]-piperidine was prepared in accordance with Example 1. M.p. 205-208°C, $[\alpha]_D^{20}$ -5.20°

Example 6. (-)N-propyl-3-[3-p-isopropylcarbamoyloxy)phenyl]piperidine was prepared in accordance with Example 1. M.p. 184-185°C, $[\alpha]_D^{20}$ = -6.91°

Example 7. (-)N-propyl-3-[3-(p-tert.butylcarbamoyloxy)phenyl]piperidine was prepared in accordance with Example 1. M.p. 179-181°C $[\alpha]_D^{20}$ = -3.05°

Example 8. (-)N-propyl-3-[3-(2-chloro-6-methylphenylcarbamoyloxy)p-phenyl]piperidine was prepared in accordance with Example 1. $[\alpha]_D^{20}$ -4.43°

The following examples illustrate how the compounds of the present invention may be included into pharmaceutical preparations.

Example P1. Preparation of soft gelatine capsules.

500 g of active substance are mixed with 500 g of corn oil, whereupon the mixture is filled in soft gelatine capsules, each capsule containing 100 mg of the mixture (i.e. 50 mg of active substance).

Example P2. Preparation of tablets.

0.5 kg of active substance are mixed with 0.2 kg of silicic acid of the trade mark Aerosil. 0.45 kg of potato starch and 0.5 kg of lactose are mixed therewith and the mixture is moistened with a starch paste prepared from 50 g of potato starch and distilled water, whereupon the mixture is granulated through a sieve. The granulate is dried and sieved, whereupon 20 g of magnesium stearate are mixed into it. Finally the mixture is pressed into tablets each weighing 172 mg.

Example P3. Preparation of a syrup.

100 g of active substance are dissolved in 300 g of 95% ethanol, whereupon 300 g of glycerol, aroma and colouring agents (q.s.) and 1000 ml of water are mixed therein. A syrup is obtained.

Example P4. Preparation of an injection solution.

Active substance (hydrochloride) (1 g), sodiumchloride (0.8 g) and ascorbic acid (0.1 g) are dissolved in sufficient amount of distilled water to give 100 ml of solution. This solution, which contains 10 mg of active substance per ml, is used in filling ampoules, which are sterilized by heating at 120°C for 20 minutes.

The compounds of the invention are believed to exert their main activity after metabolism to the corresponding compound of formula II above. Thus the compounds of the invention are believed to be prodrugs or bioprecursors of said compound of formula II. The compounds of the invention possess an improved oral absorption as compared with the compound of formula II and other previously described compounds.

Best mode of carrying out the invention

The compound of Example 1 and its salts, processes for preparing said compound and methods of employing said compound in therapy represent the best mode of carrying out the invention.

CLAIMS

1. A compound of the formula

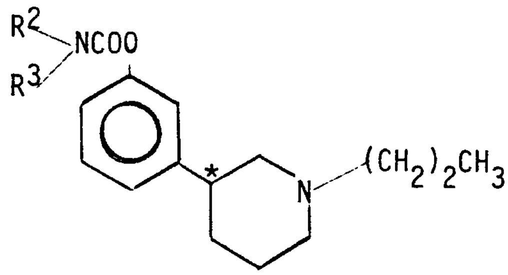

I

wherein $R^2$ is n-propyl, isopropyl, tert. butyl or one of the groups

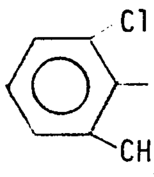 , 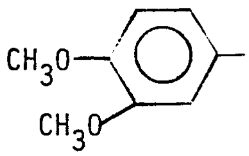 , 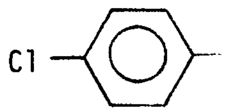

and $R^3$ is hydrogen; or $R^2$ and $R^3$ are each a n-propyl, isopropyl or tert. butyl group; or $R^2$ and $R^3$ together form the group $-(CH_2)_5-$ , as the base or a pharmaceutically acceptable acid addition salt thereof.

2. The compound according to claim 1 N-propyl-3-[3-(propylcarbamoyloxy)-phenyl]piperidine, as the base or a pharmaceutically acceptable acid addition salt thereof.

3. The compound according to claim 1 N-propyl-3-[3-(diisopropyl-carbamoyloxy)phenyl]piperidine, as the base or a pharmaceutically acceptable acid addition salt thereof.

4. The compound according to claim 1 N-propyl-3(3-piperidinecarbonyloxy-phenyl)piperidine, as the base or a pharmaceutically acceptable acid addition salt thereof.

5. The compound according to claim 1 N-propyl-3-[3-(3,4-dimethoxyphenyl-carbamoyloxy)phenyl]piperidine, as the base or a pharmaceutically acceptable acid addition salt thereof.

6. The compound according to claim 1 N-propyl-3-[3-(p-chlorophenyl-carbamoyloxy)phenyl]piperidine, as the base or a pharmaceutically acceptable acid addition salt thereof.

7. The compound according to claim 1 N-propyl-3-[3-(p-isopropylcarbamoyloxy)phenyl]piperidine, as the base or a pharmaceutically acceptable acid addition salt thereof.

8. The compound according to claim 1 N-propyl-3-[3-(p-tert.butylcarbamoyloxy)phenyl]piperidine, as the base or a pharmaceutically acceptable acid addition salt thereof.

9. The compound according to claim 1 N-propyl-3-[3-(2-chloro-6-methylphenylcarbamoyloxy)phenyl]piperidine, as the base or a pharmaceutically acceptable acid addition salt thereof.

10. A compound according to one or more of the preceding claims having the S configuration at the asymmetric carbon atom (*)

11. A process for preparation of a compound according to one or more of claims 1 to 10 wherein

a) a compound of the formula

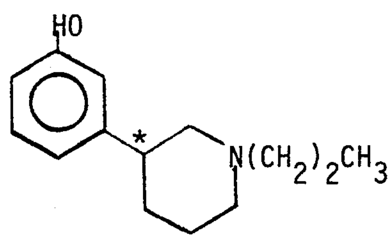

II

is converted into a compound of formula I by treating the firstmentioned compound with an appropriate carbamic acid derivative

$R^2R^3NCOX$ or aminoformaldehyde $R^2R^3NCOH$ or isocyanate $R^2NCO$ or isocyanide $R^2N \equiv C$, or

whereby X represents a leaving group such as a halogen preferably Cl or Br, or a metal sulfite $SO_3Me$ or the group $R^2R^3N$, or

b) a compound of the formula

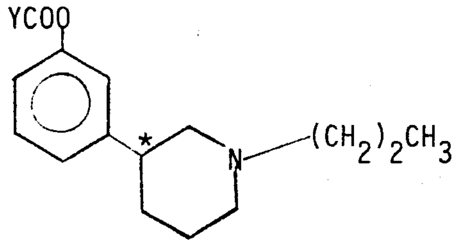

III

wherein Y is a leaving group obtainable by reaction of phosgene or a phosgene derivative with a phenol of formula II above is reacted with an amine of the formula

$R^2R^3NH$ IV

to the formation of a compound of formula I, or

c) a compound of the formula

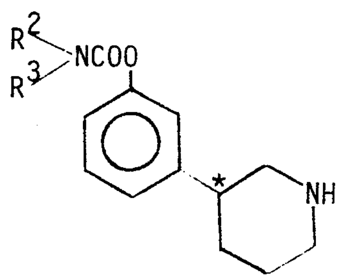

V

is converted into a compound of formula I by alkylation of the nitrogen atom with an appropriate alkylating agent, whereupon optionally a base obtained is converted to a pharmaceutically acceptable acid addition salt or a salt obtained is converted to the base or to a different, pharmaceutically acceptable acid addition salt.

12. A process for preparing a compound of the formula

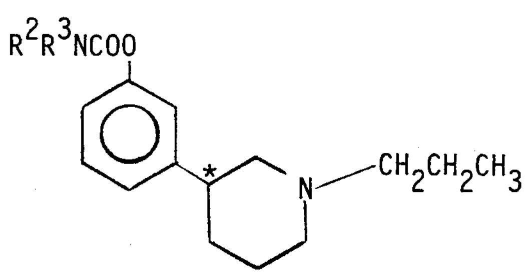

VI

wherein $R^2$ is hydrogen, an alkyl group having 1-5 carbon atoms, a phenethyl, benzyl or phenyl group which may be mono- or disubstituted

in the aromatic part with a methyl, methoxy, hydroxy, nitro or cyano group or a halogen, $R^3$ is H, an alkyl group having 1 to 5 carbon atoms or a phenyl group or $R^2$ and $R^3$ together with the nitrogen atom form a 5, 6 or 7 membered ring that may contain 1 to 3 double bonds and/or 1 or 2 further heteroatoms selected from N, O and S, wherein

a) a compound of the formula

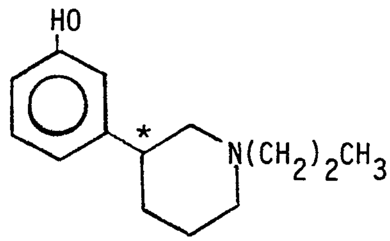

II

is converted into a compound of formula I by treating the firstmentioned compound with an appropriate carbamic acid derivative $R^2R^3NCOX$ or aminoformaldehyde $R^2R^3NCOH$ or isocyanide $R^2N{\rightleftharpoons}C$, or

whereby X represents a metal sulfite $SO_3Me$ or the group $R^2R^3N$, or

b) a compound of the formula

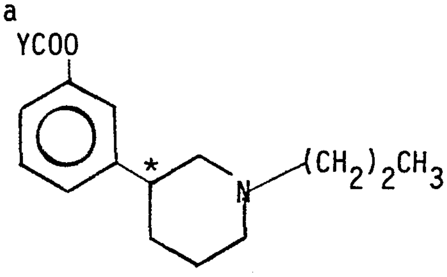

III

wherein Y is a leaving group obtainable by reaction of phosgene or a phosgene derivative with a phenol of formula II above is reacted with an amine of the formula

$$R^2R^3NH \qquad IV$$

to the formation of a compound of formula VI, or whereupon optionally a base obtained is converted to a pharmaceutically acceptable acid addition salt or a salt obtained is converted to the base or to a different, pharmaceutically acceptable acid addition salt.

13. A pharmaceutical preparation comprising as an active ingredient a compound according to one or more of claims 1 to 10, in conjunction with a pharmaceutically acceptable carrier.

14. A method of treatment of disorders in the central nervous system, comprising administering to a host in need of treatment a therapeutically effective amount of a compound according to one or more of claims 1 to 10.

15. A compound, processes, a pharmaceutical preparation and a method of treatment as claimed in any of claims 1-14, and substantially as described.